# EUROPEAN PATENT SPECIFICATION

(11) **EP 0 629 671 B1**
(45) Date of publication and mention of the grant of the patent: **27.03.2002**
(21) Application number: 94109036.7
(22) Date of filing: 13.06.1994
(51) Int. Cl.: C09D 9/00, C11D 7/50, C11D 7/26, A61K 7/06, A61K 7/48, A01N 25/02, C23G 5/032, G03F 7/42

(54) **Solvent composition**
Lösemittelzusammensetzung
Composition de solvant

(30) Priority: 15.06.1993 JP 16737493; 22.06.1993 JP 17360693; 17.09.1993 JP 25368893; 24.12.1993 JP 34605693; 11.03.1994 JP 10345794
(43) Date of publication of application: 21.12.1994
(60) Divisional application: 00104422.1
(73) Proprietor: Mitsubishi Rayon Co., Ltd., Tokyo 108-8506 (JP)
(72) Inventor: Takayanagi, Yasuyuki, c/o Nitto Chem. Ind. Co. Ltd, Yokohama-shi, Kanagawa (JP); Endou, Satoshi, c/o Nitto Chem. Ind. Co., Ltd., Yokohama-shi, Kanagawa (JP); Sugama, Naoki, c/o Nitto Chem. Ind. Co., Ltd., Yokohama-shi, Kanagawa (JP)
(74) Representative: Hansen, Bernd, Dr. Dipl.-Chem.

(56) References cited:
- US-A- 3 368 943
- DATABASE WPI Section Ch, Week 7935 Derwent Publications Ltd., London, GB; Class D23, AN 79-64034B & JP-A-54 092 635 ( KYOWA KK) , 23 July 1979

## Description

### FIELD OF THE INVENTION

This invention relates to the use of a composition as a solvent, and more particularly to a composition suitable for use as a solvent or an assistant in paints and varnishes, coatings, adhesives, printing inks, cleaning agents, agricultural chemicals, and cosmetics.

### BACKGROUND OF THE INVENTION

Solvents have played an important role in the development of the chemical industry. In particular, the importance of solvents in the fields of coating compositions, adhesives and printing inks has been ever increasing, with the recent remarkable development in the plastics industry. Coating compositions, adhesives, printing inks, etc. are generally used in the form of a solution in a solvent for assuring ease in handling on use, and for uniformly and intimately applying a high polymer base to a substrate. To this effect, it is very important for the solvent to have moderate volatility, while retaining sufficient dissolving power for the high polymer base and uniform coating properties, as well as ease in handling. In other words, the quality of coating compositions, adhesives, and printing inks largely depends on the choice of solvent.

Glycol ether type cellosolves, especially Cellosolve acetate (ethylene glycol monoethyl ether acetate), have been used for their excellent properties as solvents for cellulose resins, epoxy resins, acrylic resins, vinyl resins (e.g., vinyl acetate resins and vinyl chloride resins), alkyd resins, and polyester resins which are commonly used in the fields of coating compositions, adhesives and printing inks. In recent years, however, demand for safety of chemical substances has been increasing from the standpoint of environmental pollution. In this regard, use of Cellosolve acetate is strictly limited because of its toxicity, and the Japanese Industrial Safety and Health Law laid down criteria for controlling the working environment concentration thereof.

Intensive studies have therefore been directed to development of a solvent which can be a satisfactory substitute for Cellosolve acetate in terms of dissolving power, and yet gives rise to no safety problem. For example, ethyl lactate, propylene glycol monomethyl ether acetate, methoxypropanol, and ethyl β-ethoxypropionate have been studied as promising alternatives. However, they are not always satisfactory in dissolving power, safety, smell, and ease in handling. Of these alternative solvents, ethyl lactate, which is permitted as a food additive, seems the most preferred from the standpoint of safety, but it is not deemed to have sufficient dissolving power for high polymers and various additives. While alkyl β-alkoxypropionates, such as methyl β-methoxypropionate and ethyl β-ethoxypropionate, appear to be the most preferred from the viewpoint of dissolving power, they are still unsatisfactory in terms of dissolving power for high polymers or various additives, and in volatility after application. A mixture of methyl β-methoxypropionate and ethyl β-ethoxypropionate has been proposed as a solvent with improved physical properties, as disclosed in JP-A-3-284651 (the term "JP-A" as used herein means an "unexamined published Japanese patent application"). However, preparation of mixture involves a complicated operation, and is not suitable for industrial use. Thus, a practical solvent equal to Cellosolve acetate in performance has not yet been developed.

Besides the aforementioned applications, solvents are used in cleaning agents for removing oils, such as cutting oil, process oil, anti-corrosive oil, lubricating oil, grease and pitch, solder fluxes, inks, and liquid crystals.

Solvent-solubility of inks widely varies depending on their kind, for example, the kind of the base polymer or the hardening mechanism, such as ultraviolet-curing, heat-curing or hardening by evaporation. Therefore, an ink remover is must have a strong dissolving power to be applicable to any kind of ink.

In the production of liquid crystal displays, it is necessary to form a high-density electrode pattern on a glass substrate so as to make a fine display. However, it is not easy to keep dense lines of an electrode pattern insulated from each other. Existence of even a trace amount of a contaminant on the substrate causes display defects due to insufficient insulation or burnout due to continuous galvanic corrosion. This ultimately results in destruction of the display function. In particular, when a liquid crystal is injected into a liquid crystal cell, the liquid crystal adheres to unnecessary parts of the cell through capillary action. The adhered unnecessary liquid crystal, if left as such, will fail to provide a clear display image, and also contaminants in the air are taken up by dissolution, which tends to cause insufficient insulation. Therefore, the adhered unnecessary liquid crystal must be removed with a cell cleaner, but it is very difficult to completely remove the liquid crystal which has entered narrow gaps.

For these uses, solvents mainly comprising halogen type solvents, such as Freon 113 (1,1,2-trichloro-1,2,2- trifluoroethane), methyl chloroform (1,1,1-trichloroethane), and trichloroethylene, have been in wide use. In particular, Freon 113 has been used extensively because of its nonflammability, low toxicity, excellent stability, and dissolving power selective for various kinds of contaminants with no corrosion of metals, plastics or elastomers. However, because Freon 113 and methyl chloroform rise up to the stratosphere and destroy the ozonosphere, which eventually leads to induction of cancer of skin, their use has been severely restricted. Use of trichloroethylene is also now been restricted because it is suspected as being carcinogenic.

Accordingly, intensive studies have been conducted in order to secure a cleaning agent which will take the place of Freons, showing a cleaning action equal to the Freon 113, without entertaining a fear of destruction of the ozonosphere. For example, a number of substitutes for Freons have been proposed to date, including a composition mainly comprising 1,2-difluoroethane (see JP-A-1-132694), a mixture of 1,1-dichloro-2,2,2-trifluoroethane and dimethoxymethane (see JP-A-2-178396, corresponding to US Patent 5,068,051), and a composition mainly comprising hexafluorobenzene (see JP-A-3-167298). However, none of these solvent compositions offers a complete solution to the above-mentioned problems, i.e., they do not compare with Freon 113 in terms of performance. In addition, there is a movement to restrict use of these halogen type solvents.

On the other hand, cleaning agents for removal of fats and oils, i.e., degreasing agents, which are highly safe to humans and which do not cause environmental destruction have been proposed. For example, a composition mainly comprising a nonionic surface active agent and an alkyl lactate (see JP-A-4-68088), a composition mainly comprising a nonionic surface active agent and an adipic ester (see JP-A-4-59985), a composition mainly comprising a nonionic surface active agent and a polyalkylene glycol dialkyl ether (see JP-A-4-59984), a composition mainly comprising a nonionic surface active agent and N-methylpyrrolidone, etc. (see JP-A-4-68094), a composition mainly comprising a nonionic surface active agent and a glycerin acetate compound (see JP-A-4-68092), and a composition mainly comprising an alcohol and a fatty acid ester (see JP-A-4-68090) have been proposed. Although an alkyl lactate, N-methylpyrrolidone, etc. are highly safe solvents in terms of low toxicity, and do not cause environmental destruction or accumulate in the environment, they have insufficient dissolving power for fats and oils when used alone, as is obvious from the Comparative Examples given in the aforementioned patent publications. They essentially need a combined use of a detergent aid, such as a surface active agent, for application as a degreasing agent.

A solvent composition mainly comprising an alkyl lactate which is highly safe to humans and does not cause environmental destruction has been proposed as a cleaning agent for removing inks, i.e., an ink remover, as disclosed in JP-A-3-41170. While an alkyl lactate is a highly safe solvent in terms of low toxicity, and does not cause environmental destruction or accumulate in the environment, it is still unsatisfactory as an ink remover due to insufficient dissolving power for high polymer-based inks.

As discussed above, it has been desired in the art to develop a solvent system which substitutes for Cellosolve acetate, Freon 113, methyl chloroform, etc., while exhibiting a high dissolving power for high polymers, fats and oils, solder fluxes, liquid crystals, agricultural chemicals, cosmetics, and various compounding additives, without giving rise to safety problems.

US-A-3 368 943 reveals the use of alkyl α-alkoxyisobutyrates (e.g. isobutyl-α-methoxyisobutyrate) in solvent compositions in an amount of 0.0005% to 0.25%. Thereby the alkyl α-alkoxyisobutyrates fulfill the function of a perfume.

DATABASE WPI, Section Ch, Week 7935, Derwent Publications Ltd., London , AN 79-640348, Abstract, discloses the use of alkyl β-alkoxyisobutyrates in solvent compositions in order to fulfill the function of a perfume.

EP 0 211 667 A2 describes a radiation-sensitive resin composition comprising a solution of an alkali-soluble resin and a radiation-sensitive compound in a solvent comprising a monooxymonocarboxylic acid ester. The composition has a high storage stability and is suited for use as a resist for making integrated circuits.

### SUMMARY OF THE INVENTION

An object of the present invention is to provide a solvent composition freed of the drawbacks of conventional solvents, such as Cellosolve acetate, Freon 113 and methyl chloroform, comprising a solvent system which is of low toxicity and harmless to humans, has high dissolving power, does not produce environment destructive substances, does not give off offensive odor, and has a relatively high boiling point indicative of safety and ease in handling.

As a result of extensive research into a solvent composition having the above-described favorable properties, the present inventors have found that the object of the present invention can be met by an alkyl β-alkoxyisobutyrate. The present invention has been completed based on this finding.

The present invention provides the use of a composition containing, as an active component, at least one alkyl β-alkoxyisobutyrate represented by formula (I): 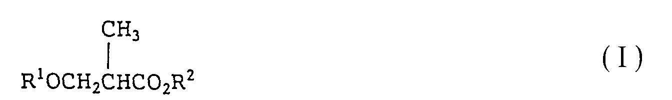 wherein R¹ and R² each represent an alkyl group having from 1 to 4 carbon atoms, in an amount of at least 5 % by weight as a solvent.

### DETAILED DESCRIPTION OF THE INVENTION

The oxyisobutyric acid ester represented by the formula (I) which can be used in the present invention is available as disclosed, for example, in EP-A-429800.

The composition of the present invention for the use as a solvent essentially contains an alkyl oxyisobutyrate of formula (I). The alkyl oxyisobutyrates of formula (I) are alkyl β-alkoxyisobutyrates, such as methyl β-methoxyisobutyrate, ethyl β-methoxyisobutyrate, methyl β-ethoxyisobutyrate, ethyl β-ethoxyisobutyrate, methyl β-isopropoxyisobutyrate, ethyl β-isopropoxyisobutyrate, butyl β-isopropoxyisobutyrate, methyl β-butoxyisobutyrate ethyl β-butoxyisobutyrate, and butyl β-butoxyisobutyrate. From the standpoint of dissolving power and volatility, methyl β-methoxyisobutyrate, ethyl β-methoxyisobutyrate, methyl β-ethoxyisobutyrate, ethyl β-ethoxyisobutyrate, methyl β-isopropoxyisobutyrate, methyl β-butoxyisobutyrate, ethyl β-butoxyisobutyrate, and butyl β-butoxyisobutyrate, are preferred.

The composition of the present invention for the use as a solvent is also useful as a degreasing agent, an ink remover, a flux remover, a liquid crystal cell cleaner or a resist stripper.

Alkyl oxyisobutyrates of formula (I) which are particularly useful as a degreasing agent include methyl β-methoxyisobutyrate, methyl β-ethoxyisobutyrate, ethyl β-ethoxyisobutyrate,methyl β-isopropoxyisobutyrate, methyl β-butoxyisobutyrate, ethyl β-butoxyisobutyrate, and butyl β-butoxyisobutyrate. Alkyl oxyisobutyrates of formula (I) which are particularly useful as an ink remover include methyl β-methoxyisobutyrate, ethyl β-methoxyisobutyrate, methyl β-ethoxyisobutyrate, ethyl β-ethoxyisobutyrate, methyl β-butoxyisobutyrate, ethyl β-butoxyisobutyrate, and butyl β-butoxyisobutyrate. Alkyl oxyisobutyrates of formula (I) which are particularly useful as a flux remover include methyl β-methoxyisobutyrate, ethyl β-methoxyisobutyrate, methyl β-ethoxyisobutyrate, ethyl β-ethoxyisobutyrate, methyl β-isopropoxyisobutyrate, methyl β-butoxyisobutyrate, ethyl β-butoxyisobutyrate, and butyl β-butoxyisobutyrate.

Alkyl oxyisobutyrates of formula (I) which are particularly useful as a liquid crystal cell cleanser include methyl β-methoxyisobutyrate, ethyl β-methoxyisobutyrate, methyl β-ethoxyisobutyrate, ethyl β-ethoxyisobutyrate, methyl β-isopropoxyisobutyrate, methyl β-butoxyisobutyrate, ethyl β-butoxyisobutyrate, and butyl β-butoxyisobutyrate. Alkyl oxyisobutyrates of formula (I) which are particularly useful as a resist stripper include methyl β-methoxyisobutyrate, ethyl β-methoxyisobutyrate, methyl β-ethoxyisobutyrate, ethyl β-ethoxyisobutyrate, methyl β-isopropoxyisobutyrate, methyl β-butoxyisobutyrate, ethyl β-butoxyisobutyrate,and butyl β-butoxyisobutyrate.

The alkyl β-alkoxyisobutyrates represented by formula (I) may be used either individually or in combination of two or more thereof.

The alkyl β-alkoxyisobutyrates are compatible with other general organic solvents, such as alcohols, esters, ketones, amides, and aromatic hydrocarbons. They exhibit markedly excellent dissolving power for a wide range of high polymers, which include natural resins, such as cellulose resins, and synthetic resins, such as epoxy resins, acrylic resins, vinyl resins (e.g., vinyl acetate resins and vinyl chloride resins), alkyd resins, polyester resins, and novolak resins, as well as general hydrocarbon-based fats and oils. Accordingly, the oxyisobutyric esters of the present invention not only serve as a solvent by themselves, but they also exhibit excellent performance as a diluent or an auxiliary solvent for other organic solvents. Thus, they can be formulated into a solvent composition together with other organic solvents. The proportion of the oxyisobutyric ester in the solvent composition is not less than 5% by weight, and preferably not less than 10% by weight, in order to take full advantage of the safety and dissolving power of the oxyisobutyric ester.

The other solvents with which the oxyisobutyric esters of the present invention may be combined are not particularly limited and include water, alcohols, ethers, esters, ketones, amides, aliphatic hydrocarbons, and aromatic hydrocarbons. Suitable examples of the other solvents are water, methanol, ethanol, isopropyl alcohol, butanol, methyl isobutyl carbinol, heptanol, octanol, nonanol, 3-methylbutanol, propylene glycol, 3-methoxybutanol, 3-methyl-3-methoxybutanol, 3,5,5-trimethyl-1-hexanol, 2-ethyl-1-hexanol, cyclohexanol, benzyl alcohol, acetone, methyl ethyl ketone, methyl isobutyl ketone, cyclohexanone, isophorone, pyrrolidone, N-methylpyrrolidone, dimethylformamide, dimethylacetamide, dimethyl sulfoxide, sulfolane, methyl acetate, ethyl acetate, butyl acetate, amyl acetate, 3-methoxybutyl acetate, 3-methyl-3-methoxybutyl acetate, methyl lactate, ethyl lactate, butyl lactate, methyl 3-methoxypropionate, ethyl 3-ethoxypropionate, 2-ethylhexyl acetate, cyclohexyl acetate, benzyl acetate, dibenzyl ether, nitromethane, nitroethane, acetonitrile, γ-butyrolactone, propylene glycol monomethyl ether acetate, toluene, xylene, hexane, and cyclohexane. Preferred of them are water, methanol, ethanol, isopropyl alcohol, butanol, heptanol, octanol, 3-methylbutanol, 3-methoxybutanol, 3-methyl-3-methoxybutanol, 3,5,5-trimethyl-1-hexanol, 2-ethyl-1-hexanol, cyclohexanol, benzyl alcohol, acetone, methyl ethyl ketone, methyl isobutyl ketone, cyclohexanone, isophorone, N-methylpyrrolidone, dimethylformamide, methyl acetate, ethyl acetate, butyl acetate, amyl acetate, 3-methoxybutyl acetate, 3-methyl-3-methoxybutyl acetate, methyl lactate, ethyl lactate, butyl lactate, methyl 3-methoxypropionate, ethyl 3-ethoxypropionate, 2-ethylhexyl acetate, cyclohexyl acetate, benzyl acetate, dibenzyl ether, acetonitrile, propylene glycol monomethyl ether acetate, toluene, and xylene.

Solvents which are particularly preferred to provide a degreasing agent include isopropyl alcohol, butanol, heptanol, octanol, 3-methylbutanol, 3-methoxybutanol, 3-methyl-3-methoxybutanol, 3,5,5-trimethyl-1-hexanol, 2-ethyl-1-hexanol, cyclohexanol, benzyl alcohol, acetone, methyl ethyl ketone, methyl isobutyl ketone, cyclohexanone, isophorone, N-methylpyrrolidone, dimethylformamide, methyl acetate, ethyl acetate, butyl acetate, amyl acetate, 3-methoxybutyl acetate, 3-methyl-3-methoxybutyl acetate, methyl lactate, ethyl lactate, butyl lactate, methyl 3-methoxypropionate, ethyl 3-ethoxypropionate, 2-ethylhexyl acetate, cyclohexyl acetate, benzyl acetate, dibenzyl ether, acetonitrile, propylene glycol monomethyl ether acetate, toluene, and xylene.

Solvents which are particularly useful to provide an ink remover include water, ethanol, isopropyl alcohol, butanol, heptanol, octanol, 3-methylbutanol, 3-methoxybutanol, 3-methyl-3-methoxybutanol, 3,5,5-trimethyl-1-hexanol, 2-ethyl-1-hexanol, cyclohexanol, benzyl alcohol, acetone, methyl ethyl ketone, methyl isobutyl ketone, cyclohexanone, isophorone, N-methylpyrrolidone, dimethylformamide, methyl acetate, ethyl acetate, butyl acetate, amyl acetate, 3-methoxybutyl acetate, 3-methyl-3-methoxybutyl acetate, methyl lactate, ethyl lactate, butyl lactate, methyl 3-methoxypropionate, ethyl 3-ethoxypropionate, 2-ethylhexyl acetate, cyclohexyl acetate, benzyl acetate, dibenzyl ether, acetonitrile, propylene glycol monomethyl ether acetate, toluene, and xylene.

Solvents which are particularly useful to provide a flux remover include water, methanol, ethanol, isopropyl alcohol, butanol, heptanol, octanol, 3-methylbutanol, 3-methoxybutanol, 3-methyl-3-methoxybutanol, 3,5,5-trimethyl-1-hexanol, 2-ethyl-1-hexanol, cyclohexanol, benzyl alcohol, acetone, methyl ethyl ketone, methyl isobutyl ketone, cyclohexanone, isophorone, N-methylpyrrolidone, dimethylformamide, methyl acetate, ethyl acetate, butyl acetate, amyl acetate, 3-methoxybutyl acetate, 3-methyl-3-methoxybutyl acetate, methyl lactate, ethyl lactate, butyl lactate, methyl 3-methoxypropionate, ethyl 3-ethoxypropionate, 2-ethylhexyl acetate, cyclohexyl acetate, benzyl acetate, dibenzyl ether, acetonitrile, propylene glycol monomethyl ether acetate, nitromethane, nitroethane, toluene, and xylene.

Solvents which are particularly useful to provide a liquid crystal cell cleaner include isopropyl alcohol, butanol, heptanol, octanol, 3-methylbutanol, 3-methoxybutanol, 3-methyl-3-methoxybutanol, 3,5,5-trimethyl-1-hexanol, 2-ethyl-1-hexanol, cyclohexanol, benzyl alcohol, acetone, methyl ethyl ketone, methyl isobutyl ketone, cyclohexanone, isophorone, N-methylpyrrolidone, dimethylformamide, methyl acetate, ethyl acetate, butyl acetate, amyl acetate, 3-methoxybutyl acetate, 3-methyl-3-methoxybutyl acetate, methyl lactate, ethyl lactate, butyl lactate, methyl 3-methoxypropionate, ethyl 3-ethoxypropionate, 2-ethylhexyl acetate, cyclohexyl acetate, benzyl acetate, dibenzyl ether, acetonitrile, propylene glycol monomethyl ether acetate, nitromethane, nitroethane, toluene, and xylene.

Solvents which are particularly useful to provide a resist stripper include ethanol, isopropyl alcohol, butanol, 3-methylbutanol, 3-methoxybutanol, 3-methyl-3-methoxybutanol, 2-ethyl-1-hexanol, cyclohexanol, benzyl alcohol, acetone, methyl ethyl ketone, methyl isobutyl ketone, cyclohexanone, isophorone, N-methylpyrrolidone, dimethylformamide, methyl acetate, ethyl acetate, butyl acetate, amyl acetate, 3-methoxybutyl acetate, 3-methyl-3-methoxybutyl acetate, methyl lactate, ethyl lactate, butyl lactate, methyl 3-methoxypropionate, ethyl 3-ethoxypropionate, 2-ethylhexyl acetate, cyclohexyl acetate, benzyl acetate, dibenzyl ether, acetonitrile, propylene glycol monomethyl ether acetate, toluene, and xylene.

These organic solvents and water may be used either individually or in combination of two or more thereof. A combined use of the organic solvent or water makes it possible to appropriately improve or modify the cleaning properties, safety, ease in handling, and the like of the solvent composition of the present invention.

Since the alkyl oxyisobutyrates of the present invention have a high boiling point and a relatively low rate of evaporation, they are useful as high-boiling solvents. When compounded into mixed solvent systems, they bring about improvements in performance properties and workability of coating compositions, adhesives, ink compositions, etc., such as spreadability and smoothness of a coating film and effects on fusion of resins.

The content of the alkyl oxyisobutyrate in the solvent composition is not less than 5% by weight, and preferably not less than 10% by weight, depending on the use.

When used as a cleaning agent, the cleansing action of the solvent composition may be improved, if desired, by using a surface active agent, such as a nonionic surface active agent (e.g., polyalkyleneoxides and alkanolamides), a anionic surface active agent (e.g., alkyl(aryl)sulfonic acids and alkylphosphonic acids) or a cationic surface active agent (e.g., long chain amines and quaternary ammonium salts), an acidic compound, or a basic compound in combination. The typical example of the surface active agent includes polyoxyethylene dodecyl ether, sodium dodecylbenzenesulfonic acid and trimethylbenzylammonium chloride. The typical example of the acidic compound includes acetic acid, α-methoxyisobutyric acid, β-methoxyisobutyric acid, α-hydroxyisobutyric acid and methacrylic acid. The typical example of the basic compound includes triethylamine and triethanolamine. The surface active agent, acidic compound and basic compound is preferably used in an amount of from about 0.01 to 30% by weight, more preferably from 0.1 to 10% by weight, based on the total solvent composition.

When used as a resist stripper, the solvent composition may further contain a stripping accelerator, such as benzenesulfonic acid, toluenesulfonic acid, xylenesulfonic acid, phenolsulfonic acid, and alkylbenzenesulfonic acids, e.g., methyl-, propyl-, heptyl-, octyl-, decyl- or dodecylbenzenesulfonic acid. The stripping accelerator is usually used in an amount of from about 5 to 30% by weight, preferably 7 to 20% by weight, based on the total solvent composition. To improve stripping properties, the composition may furthermore contain a surface active agent, such as a nonionic surface active agent, anionic surface active agent or a cationic surface active agent, an acidic compound, or a basic compound.

The oxyisobutyric esters of the present invention have very high dissolving power for various organic compounds, including high polymers and naturally-occurring compounds. On the other hand, the alkyl oxyisobutyrates of the present invention do not have risk of explosion at an ordinal temperature, because their ignition point is 40°C or more. In addition, the acute toxicity (median lethal dose (LD₅₀); rat, oral) of the alkyl oxyisobutyrates is 2000mg/kg or more. Therefore, they are also useful as an assistant for diluting solvents for agricultural chemicals or as a solvent or an assistant for cosmetics.

The photoresists to which the resist stripper of the present invention is applicable are not limited at all. That is, the resist stripper of the invention is useful for removal of any of positive or negative resists for optical alignment, resists for far ultraviolet light alignment, and resists for X-ray or electron beam alignment. Main materials known for these resists include novolak resins, cyclized rubbers, polysilicic acid, (meth)acrylic resins, (meth)acrylic acid copolymers, and polyhydroxystyrene. The resist stripper of the present invention is effective on any of these high polymers.

The present invention will now be illustrated in greater detail with reference to the Examples in view of the Comparative Examples, but the present invention should not be construed as being limited thereto. All of the mixing ratios of the solvents are given by weight unless otherwise indicated.

### EXAMPLE 1

The compatibility of the alkyl oxyisobutyrates of the present invention with other solvents was examined as follows.

Methyl β-methoxyisobutyrate, or a 30/70 mixture of methyl β-methoxyisobutyrate and methyl α-hydroxyisobutyrate, and the solvents shown in Table 1 below were mixed in a mixing ratio of 1/1 by volume in an Erlenmeyer flask, and the mixture was allowed to stand still at room temperature. The degree of compatibility was visually observed and rated as follows. The results obtained are shown in Table 1.
- A: Completely uniformly mixed.
- B: White turbidity observed.
- C: Separated into two phases.

### COMPARATIVE EXAMPLE 1

The compatibility of Cellosolve acetate (ethylene glycol monoethyl ether acetate; hereinafter abbreviated as "ECA") with the solvent shown in Table 1 was examined in the same manner as in Example 1. The results obtained are shown in Table 1 below.

**TABLE 1**

| Solvent | Example 1 | | Compar. Example 1 |
|---|---|---|---|
| | β-MBM¹⁾ | β-MBM/α-HBM²⁾ | ECA |
| Water | C | B | C |
| Methanol | A | A | A |
| Ethanol | A | A | A |
| Isopropyl alcohol | A | A | A |
| Ethyl acetate | A | A | A |
| Acetone | A | A | A |
| dimethylformamide | A | A | A |
| Acetonitrile | A | A | A |
| Benzene | A | A | A |
| Toluene | A | A | A |
| Xylene | A | A | A |
| n-Hexane | A | A | A |
| Cyclohexane | A | A | A |
| Cyclohexanone | A | A | A |
| Cyclohexanol | A | A | A |

| | | | |
|---|---|---|---|
| Note: 1): Methyl β-methoxyisobutyrate | | | |
| 2): β-MBM/α-HBM = 30/70 | | | |

### EXAMPLE 2

The rate of evaporation of the alkyl oxyisobutyrates of the present invention was measured as follows.

The solvents shown in Table 2 below weighing 0.75 g, were uniformly spread on a disc of No. 4 filter paper having a diameter of 95 mm. The coated filter paper was allowed to stand on a testing stand at 25°C with a covering over it. The sample was weighed at given time intervals to determine the evaporation loss, from which the rate of evaporation was calculated. The results obtained, expressed relatively taking the rate of evaporation of butyl acetate as a standard (100), are shown in Table 2 below.

**TABLE 2**

| Solvent | Relative Rate of Evaporation |
|---|---|
| Butyl acetate | 100 (standard) |
| Methyl β-methoxyisobutyrate | 44 |
| Ethyl β-ethoxyisobutyrate | 16 |

### COMPARATIVE EXAMPLE 2

The rate of evaporation of the conventional solvents shown in Table 3 below was determined in the same manner as in Example 2. The results obtained are shown in Table 3 below.

**TABLE 3**

| Solvent | Relative Rate of Evaporation |
|---|---|
| Butyl acetate | 100 (standard) |
| ECA | 22 |
| Ethyl lactate | 23 |
| Methyl β-methoxypropionate | 35 |

### EXAMPLE 3

The dissolving power of methyl β-methoxyisobutyrate for various resins was evaluated as follows.

In a 100 mℓ-volume Erlenmeyer flask was put 2.0 g of the resins shown in Table 4 below, and 20 mℓ of methyl β-methoxyisobutyrate was added thereto. The mixture was kept at 25°C while stirring, and the time required for the resin to dissolve was measured. The results obtained are shown in Table 4 below.

### COMPARATIVE EXAMPLE 3

The dissolving power of ECA was evaluated in the same manner as in Example 3. The results are shown in Table 4 below.

**TABLE 4**

| Resin | Time for Dissolution (min) | |
|---|---|---|
| | Example 3 | Compar. Example 3 |
| Polyester (ER-1002, a product of Mitsubishi Rayon Co., Ltd.) | 110 | 150 |
| Polystyrene (QPX2B, a product of Denki Kagaku Kogyo K.K. | 46 | 86 |
| Methyl methacrylate-styrene copolymer (MS-300, a product of Nippon Steel Chemical Co., Ltd.) | 82 | 125 |
| Acrylonitrile-styrene copolymer (AS-30, a product of Asahi Chemical Industry Co., Ltd.) | 95 | 145 |
| Acrylic resin (Acrydic A-405, a product of Dainippon Ink and Chemicals, Inc.) | 30 | 45 |
| Phenoxy resin (PKH-H, a product of Union Carbide Corp.) | 110 | 150 |
| Polysulfone (P1800NT11, a product of Amoco Co.) | 160 | 260 |

### EXAMPLE 4

The dissolving power of the alkyl oxyisobutyrates shown in Table 5 below for an epoxy resin was evaluated.

In a 100 mℓ-volume Erlenmeyer flask was put 2.0 g of an epoxy resin (Epikote 1007, a product of Yuka Shell Epoxy Co., Ltd.), and 20 mℓ of the alkyl oxyisobutyrate was added thereto. The mixture was kept at 25°C while stirring, and the time required for the resin to dissolve was measured. The results obtained are shown in Table 5 below.

**TABLE 5**

| Solvent | Time of Dissolution (min) |
|---|---|
| Methyl β-methoxyisobutyrate | 25 |
| Ethyl β-ethoxyisobutyrate | 28 |

### COMPARATIVE EXAMPLE 4

The epoxy resin-dissolving power of ECA, ethyl lactate or methyl β-methoxypropionate was evaluated in the same manner as in Example 4. The results are shown in Table 6 below.

**TABLE 6**

| Solvent | Time of Dissolution (min) |
|---|---|
| ECA | 45 |
| Ethyl lactate | 65 |
| Methyl β-methoxypropionate | 42 |

As can be seen from Table 1, the oxyisobutyric esters of the present invention have compatibility equal or superior to the conventional general-purpose solvents, such as ECA, and thus can be used as a mixture with a broad range of other solvents.

As can be seen from Tables 2 and 3, the a rate of evaporation can arbitrarily be selected by a proper choice of the ester group. The rate of evaporation of the oxyisobutyric esters of the present invention is about 2 to 3 times as high as that of ECA or ethyl lactate, which have been widely used, proving that the oxyisobutyric esters provide a solvent system having good volatility.

As can be seen from Tables 4, 5 and 6, the oxyisobutyric esters of the present invention need a shorter time for dissolving various resins, often used in coating compositions and adhesives, than needed by the conventional general-purpose solvents. This reveals their excellent resin-dissolving power.

### EXAMPLE 5

A 50 mm long, 20 mm wide, and 2 mm thick stainless steel plate with its surface polished was uniformly coated with commercially available fats and oils, i.e., cutting oil (Daphne-Mag plus LA15, a product of Idemitsu Kosan Co., Ltd.), press oil (Unipress DP120, a product of Nippon Oil Co., Ltd.) or grease (Albania Grease 1, a product of Showa Shell Sekiyu K.K.). One hour later, the coated plate was put in a washing bottle containing the degreasing agent shown in Table 7 below, and the bottle and content was shaken at 40°C for 5 minutes. The stainless steel plate was taken out and dried in hot air. The oil or grease remaining on the plate was observed with the naked eye and under a metallurgical microscope, and the degreasing power was evaluated according to the following rating system.
- A: No residual oil or grease was observed with the naked eye or under a metallurgical microscope.
- B: Residual oil or grease was not observed with the naked eye, but observed under a metallurgical microscope.
- C: Residual oil or grease was observed with the naked eye.

The results obtained are shown in Table 7 below.

**TABLE 7**

| | Degreasing Power | | |
|---|---|---|---|
| Degreasing Agent | Cutting Oil | Press Oil | Grease |
| Methyl β-methoxyisobutyrate | A | A | A |
| Methyl β-methoxyisobutyrate/methyl α-hydroxyisobutyrate (30:70) | A | A | A |
| Ethyl β-ethoxyisobutyrate/ methyl isobutyl ketone (80:20) | A | A | A |
| Methyl β-ethoxyisobutyrate/ dimethylformamide (50:50) | A | A | A |
| Methyl β-methoxyisobutyrate/N-methylpyrrolidone/water (50:40:10) | A | A | A |
| Methyl α-methoxyisobutyrate/methyl β-methoxyisobutyrate (30:70) | A | A | A |
| Methyl β-methoxyisobutyrate/butyl acetate/octanol (50:20:30) | A | A | A |
| Methyl β-methoxyisobutyrate/ isophorone (70:30) | A | A | A |
| Methyl β-methoxyisobutyrate/amyl acetate/3-methoxybutanol (30:50:20) | A | A | A |
| Methyl β-methoxyisobutyrate/2-ethylhexanol/dibenzyl ether (50:30:20) | A | A | A |
| Methyl β-methoxyisobutyrate/2-ethylhexyl acetate/benzyl alcohol (50:30:20) | A | A | A |

### COMPARATIVE EXAMPLE 5

The conventional degreasing agents shown in Table 8 below were tested in the same manner as in Example 5. The results obtained are shown in Table 8 below.

**TABLE 8**

| | Degreasing Power | | |
|---|---|---|---|
| Degreasing Agent | Cutting Oil | Press Oil | Grease |
| 1,1,1-Trichloroethane | A | B | A |
| Freon 113 | A | C | B |
| Ethyl lactate | C | C | C |
| N-Methylpyrrolidone | B | C | C |

### EXAMPLE 6

Various commercially available inks were uniformly spread on a 50 mm long, 20 mm wide, and 2 mm thick aluminum plate with a polished surface by means of a bar coder. One hour later, the coated aluminum plate was put in a washing bottle containing the ink removers shown in Table 9 below, and the bottle and content was shaken at room temperature for 30 seconds. The plate was taken out and dried. The ink remaining on the plate was observed with the naked eye and under a metallurgical microscope, and the ink removing power was evaluated according to the following rating system.
- A: No residual ink was observed with the naked eye or under a metallurgical microscope.
- B: Residual ink was not observed with the naked eye, but observed under a metallurgical microscope.
- C: Residual ink was observed with the naked eye.

The results obtained are shown in Table 9 below.

**TABLE 9**

| | Ink Removing Power | | | |
|---|---|---|---|---|
| Ink Remover | 1* | 2* | 3* | 4* |
| Methyl β-methoxyisobutyrate | A | A | A | A |
| Methyl β-methoxyisobutyrate/methyl α-hydroxyisobutyrate (30:70) | A | A | A | B |
| Ethyl β-ethoxyisobutyrate/methyl isobutyl- ketone (80:20) | A | A | A | A |
| Methyl β-ethoxyisobutyrate/ dimethylformamide (50:50) | A | A | A | A |
| Methyl β-methoxyisobutyrate/N-methylpyrrolidone/water (50:40:10) | A | A | A | A |
| Methyl α-methoxyisobutyrate/methyl β-methoxyisobutyrate (30:70) | A | A | A | A |
| Methyl β-methoxyisobutyrate/butyl acetate/octanol (50:20:30) | A | A | A | A |
| Methyl β-methoxyisobutyrate/ isophorone (70:30) | A | A | A | A |
| Methyl β-methoxyisobutyrate/amyl acetate/3-methoxybutanol (30:50:20) | A | A | A | A |
| Methyl β-methoxyisobutyrate/2-ethylhexanol/dibenzyl ether (50:30:20) | A | A | A | A |
| Methyl β-methoxyisobutyrate/2-ethylhexyl acetate/benzyl alcohol (50:30:20) | A | A | A | A |

| | | | | |
|---|---|---|---|---|
| Note: 1*: Cellulose resin-based green ink for printing letters on instrument boards | | | | |
| 2*: Hot-setting green solder resist ink | | | | |
| 3*: UV-curing white marking ink | | | | |
| 4*: Vinyl chloride-acrylate copolymer-based white screen printing ink | | | | |

### COMPARATIVE EXAMPLE 6

The conventional ink removers shown in Table 10 below were tested in the same manner as in Example 6. The results obtained are shown in Table 10 below.

**TABLE 10**

| | Ink Removing Power | | | |
|---|---|---|---|---|
| Ink Remover | 1* | 2* | 3* | 4* |
| 1,1,1-Trichloroethane | A | B | A | C |
| Ethyl lactate | A | C | B | C |
| 1,1,1-Trichloroethane/ cyclohexanone (80:20) | A | A | A | B |
| Methyl β-methoxypropionate | B | B | A | B |

### EXAMPLE 7

A commercially available flux (Solderite flux B-111R, a product of Tamura Corporation) was uniformly applied to the entire surface of a base of a printed circuit board (a copper-clad laminate), preliminarily dried at 100°C, and baked at 220°C.

The coated base was cleaned by immersing in the flux removers shown in Table 11 below while shaking at room temperature for 5 minutes. The base was taken out, washed with water, and dried. The residual flux on the base was observed with the naked eye and under a metallurgical microscope, and rated as follows.
- A: No residual flux was observed with the naked eye or under a metallurgical microscope.
- B: Residual flux was not observed with the naked eye, but observed under a metallurgical microscope.
- C: Residual flux was observed with the naked eye.

The results obtained are shown in Table 11 below.

**TABLE 11**

| Flux Remover | Flux Removing Power |
|---|---|
| Methyl β-methoxyisobutyrate | A |
| Methyl β-methoxyisobutyrate/methyl α-hydroxyisobutyrate (30:70) | A |
| Ethyl β-ethoxyisobutyrate/acetone (50:50) | A |
| Methyl β-ethoxyisobutyrate/dimethylformamide (50:50) | A |
| Methyl β-methoxyisobutyrate/N-methylpyrrolidone/water (40:50:10) | A |
| Methyl α-methoxyisobutyrate/methyl β-methoxyisobutyrate (30:70) | A |
| Methyl β-methoxyisobutyrate/methyl lactate (30:70) | A |
| Methyl β-methoxyisobutyrate/butyl acetate/octanol (50:20:30) | A |
| Methyl β-methoxyisobutyrate/ isophorone (70:30) | A |
| Methyl β-methoxyisobutyrate/amyl acetate/3-methoxybutanol (30:50:20) | A |
| Methyl β-methoxyisobutyrate/N-methylpyrrolidone/water/acetic acid (30:50:15:5) | A |
| Methyl β-methoxyisobutyrate/2-ethylhexanol/dibenzyl alcohol (50:30:20) | A |
| Methyl β-methoxyisobutyrate/2-ethylhexyl acetate/benzyl alcohol (50:30:20) | A |

### COMPARATIVE EXAMPLE 7

The conventional flux removers shown in Table 12 below were tested in the same manner as in Example 7. The results obtained are shown in Table 12 below.

**TABLE 12**

| Flux Remover | Flux Removing Power |
|---|---|
| Freon 113 | C |
| Methyl lactate | B |

### EXAMPLE 8

A commercially available flux (Tamura F-Al-4, a product of Tamura Corporation) was uniformly applied to the entire surface of a base of a printed circuit board (a copper-clad laminate), preliminarily dried at 100°C, and baked at 220°C.

The coated base was cleaned by immersing in the flux removers shown in Table 13 below while shaking at 40°C for 5 minutes. The base was taken out, washed with water, and dried. The residual flux on the base was observed with the naked eye and under a metallurgical microscope, and rated according to the same standard as in Example 7.

The results obtained are shown in Table 13 below.

**TABLE 13**

| Flux Remover | Flux Removing Power |
|---|---|
| Methyl α-hydroxyisobutyrate/ methyl β-methoxyisobutyrate (70:30) | A |

### COMPARATIVE EXAMPLE 8

The conventional flux removers shown in Table 14 below were tested in the same manner as in Example 8. The results obtained are shown in Table 14 below.

**TABLE 14**

| Flux Remover | Flux Removing Power |
|---|---|
| Freon 113 | C |
| Methyl lactate | B |

### EXAMPLE 9

A liquid crystal cell assembled from a pair of glass plates (60 x 30 x 1 mm), with the surface having been cleaned, and spacers to have a cell gap of 10 µm was filled with a commercially available liquid crystal (ZLI-1565, a product of Merck Japan Co., Ltd.) and sealed.

The liquid crystal cell was washed by soaking in 100 mℓ of the cell cleaners shown in Table 15 below at room temperature for 10 minutes. The cell was taken out and dried in hot air. The liquid crystal remaining on the glass plates and the periphery of the cell was observed with the naked eye and a polarizing microscope, and rated as follows.
- A: No residual liquid crystal was observed with the naked eye or under a polarizing microscope.
- B: Residual liquid crystal was not observed with the naked eye, but observed under a polarizing microscope.
- C: Residual liquid crystal was observed with the naked eye.

The results obtained are shown in Table 15 below.

**TABLE 15**

| Liquid Crystal Cleaner | Liquid Crystal Removing Power |
|---|---|
| Methyl β-methoxyisobutyrate | A |
| Methyl β-methoxyisobutyrate/methyl α-hydroxyisobutyrate (30:70) | A |
| Ethyl β-ethoxyisobutyrate/methyl isobutyl ketone (80:20) | A |
| Methyl β-methoxyisobutyrate/ dimethylformamide (50:50) | A |
| Methyl β-methoxyisobutyrate/ N-methylpyrrolidone (50:50) | A |
| Methyl α-methoxyisobutyrate/methyl β-methoxyisobutyrate (30:70) | A |
| Methyl β-methoxyisobutyrate/ethyl lactate/dodecylbenzenesulfonic acid (50:40:10) | A |
| Methyl β-methoxyisobutyrate/butyl acetate/octanol (50:20:30) | A |
| Methyl β-methoxyisobutyrate/ isophorone (70:30) | A |
| Methyl β-methoxyisobutyrate/amyl acetate/3-methoxybutanol (30:50:20) | A |
| Methyl β-methoxyisobutyrate/2-ethylhexanol/dibenzyl ether (50:30:20) | A |
| Methyl β-methoxyisobutyrate/2-ethylhexyl acetate/benzyl alcohol (50:30:20) | A |

### COMPARATIVE EXAMPLE 9

The conventional cell cleaners shown in Table 16 below were tested in the same manner as in Example 9. The results obtained are shown in Table 16 below.

**TABLE 16**

| Liquid Crystal Cleaner | Liquid Crystal Removing Power |
|---|---|
| 1,1,1-Trichloroethane | B |
| Polyoxyethylene butyl ether/ethanol/water (30:35:35) | C |
| Ethyl lactate | C |
| Methyl β-methoxypropionate | B |

### EXAMPLE 10

A silicon wafer was uniformly coated with a commercially available positive photoresist for fine processing (OFPR-800, a product of Tokyo Ohka Kogyo Co., Ltd.) with a spinner (3000 rpm), pre-baked at 90°C for 30 minutes, and exposed to ultraviolet light to form a pattern. The resist was developed with an alkali developer (NMD-W, a product of Tokyo Ohka Kogyo Co., Ltd.) at 25°C for 1 minute, rinsed with pure water for 30 minutes, and post baked at 130°C for 30 minutes. Then, the resist was removed by immersing in the resist strippers shown in Table 17 below for 5 minutes while shaking, washed with water, and dried. The residual resist film was observed with the naked eye and under a metallurgical microscope, and rated as follows.
- A: No residual resist was observed with the naked eye or under a metallurgical microscope.
- B: Residual resist was not observed with the naked eye, but observed under a metallurgical microscope.
- C: Residual resist was observed with the naked eye.

The results obtained are shown in Table 17 below.

**TABLE 17**

| Resist Stripper | Resist Removing Power |
|---|---|
| Methyl β-methoxyisobutyrate | A |
| Methyl β-methoxyisobutyrate/methyl α-hydroxyisobutyrate/dodecylbenzenesulfonic acid (15:70:15) | A |
| Ethyl β-ethoxyisobutyrate/acetone (80:20) | A |
| Methyl β-ethoxyisobutyrate/dimethylformamide (50:50) | A |
| Methyl β-methoxyisobutyrate/N-methylpyrrolidone/water (50:40:10) | A |
| Methyl α-methoxyisobutyrate/methyl β-methoxyisobutyrate (30:70) | A |

### COMPARATIVE EXAMPLE 10

The conventional resist strippers shown in Table 18 below were tested in the same manner as in Example 10. The results obtained are shown in Table 18 below.

**TABLE 18**

| Resist Stripper | Resist Removing Power |
|---|---|
| ECA | C |
| Ethyl lactate | C |
| Ethyl lactate/dodecylbenzenesulfonic acid (80:20) | B |
| Methyl β-methoxypropionate | B |
| Methyl β-methoxypropionate/dodecylbenzenesulfonic acid (80:20) | A |

As described above, the solvent composition according to the present invention has excellent dissolving power, gives off no offensive smell, gives rise to no environmental problem, and is safe. Additionally, the solvent composition of the present invention has the following advantages:
1) It has an extremely high dissolving power for natural and synthetic high polymers;
2) It is freely miscible with many organic solvents;
3) It is biodegradable, non-accumulating in nature;
4) It has low toxicity, no teratogenicity, and high safety;
5) It has a relatively high boiling point and ignition point, so as to secure improved handling properties and safety; and
6) It is non-corrosive for a substrate.

The degreasing agent comprising the solvent composition of the present invention has high cleaning power. Since the degreasing agent has an extremely high dissolving power for various fats and oils as well as the above-mentioned advantages, it exhibits excellent ability in removal of fats, oils, and grease. Being water-soluble, the degreasing agent can be used in an aqueous system.

The ink remover comprising the solvent composition of the present invention has extremely high dissolving power for various types of inks, as well as the above-mentioned advantages, and therefore exhibits excellent ability in ink removal.

The flux remover comprising the solvent composition of the present invention exhibits an extremely high dissolving power for fluxes, as well as the above-mentioned advantages (1) to (6), and therefore has excellent ability in flux removal.

The liquid crystal cell cleaner comprising the solvent composition of the present invention has an extremely high dissolving power for various liquid crystals, as well as the above-mentioned advantages (1) to (6), and therefore exhibits excellent ability in removal of liquid crystals, and particularly contaminants from liquid crystal cells.

The resist stripper comprising the solvent composition of the present invention has an extremely high dissolving power for a photoresist, in addition to the above-mentioned effects (1) to (6), and exhibits excellent performance in removal of a resist.

## Claims

1. Use of a composition comprising, as an active compound, at least one alkyl β-alkoxyisobutyrate represented by the formula (I): 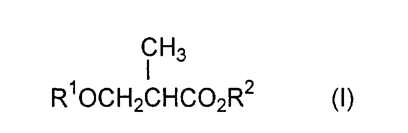 wherein R¹ and R² each represent an alkyl group having from 1 to 4 carbon atoms, in an amount of at least 5% by weight, as a solvent.

2. Use according to claim 1, wherein said composition further comprises at least one compound selected from the group consisting of water, methanol, ethanol, isopropyl alcohol, butanol, methyl isobutyl carbinol, heptanol, octanol, nonanol, 3-methylbutanol, propylene glycol, 3-methoxybutanol, 3-methyl-3-methoxybutanol, 3,5,5-trimethyl-1-hexanol, 2-ethyl-1-hexanol, cyclohexanol, benzyl alcohol, acetone, methyl ethyl ketone, methyl isobutyl ketone, cyclohexanone, isophorone, pyrrolidone, N-methylpyrrolidone, dimethylformamide, dimethylacetamide, dimethyl sulfoxide, sulfolane, methyl acetate, ethyl acetate, butyl acetate, amyl acetate, 3-methoxybutyl acetate, 3-methyl-3-methoxybutyl acetate, methyl lactate, ethyl lactate, butyl lactate, methyl 3-methoxypropionate, ethyl 3-ethoxypropionate, 2-ethylhexyl acetate, cyclohexyl acetate, benzyl acetate, dibenzyl ether, nitromethane, nitroethane, acetonitrile, γ-butyrolactone, propylene glycol monomethyl ether acetate, toluene, xylene, hexane, and cyclohexane.

3. Use according to claim 1, wherein said alkyl β-alkoxyisobutyrate is selected from the group consisting of methyl β-methoxyisobutyrate, ethyl β-methoxisobutyrate, methyl β-ethoxyisobutyrate, ethyl β-ethoxyisobutyrate, methyl β-isopropoxyisobutyrate, ethyl β-isopropoxyisobutyrate, butyl β-isopropoxyisobutyrate, methyl β-butoxyisobutyrate, ethyl β-butoxyisobutyrate and butyl β-butoxyisobutyrate.

## Patentansprüche

1. Verwendung einer Zusammensetzung, die als aktive Verbindung mindestens ein Alkyl-β-alkoxyisobutyrat, dargestellt durch die Formel (1): 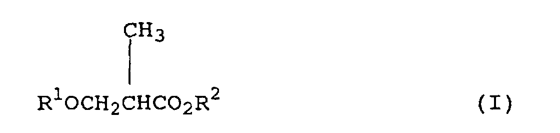 worin R¹ und R² jeweils eine Alkylgruppe mit 1 bis 4 Kohlenstoffatomen darstellen, in einer Menge von mindestens 5 Gew.% umfaßt, als Lösungsmittel.

2. Verwendung gemäß Anspruch 1, worin die genannte Zusammensetzung weiterhin mindestens eine Verbindung, ausgewählt aus der Gruppe, bestehend aus Wasser, Methanol, Ethanol, Isopropylalkohol, Butanol, Methylisobutylkarbinol, Heptanol, Octanol, Nonanol, 3-Methylbutanol, Propylenglycol, 3-Methoxybutanol, 3-Methyl-3-methoxybutanol, 3,5,5-Trimethyl-1-hexanol, 2-Ethyl-1-hexanol, Cyclohexanol, Benzylalkohol, Aceton, Methylethylketon, Methylisobutylketon, Cyclohexanon, Isophoron, Pyrrolidon, N-Methylpyrrolidon, Dimethylformamid, Dimethylacetamid, Dimethylsulfoxid, Sulfolan, Methylacetat, Ethylacetat, Butylacetat, Amylacetat, 3-Methoxybutylacetat, 3-Methyl-3-methoxybutylacetat, Methyllactat, Ethyllactat, Butyllactat, Methyl-3-methoxypropionat, Ethyl-3-ethoxypropionat, 2-Ethylhexylacetat, Cyclohexylacetat, Benzylacetat, Dibenzylether, Nitromethan, Nitroethan, Acetonitril, γ-Butyrolacton, Propylenglycolmonomethyletheracetat, Toluol, Xylol, Hexan und Cyclohexan, umfaßt.

3. Verwendung gemäß Anspruch 1, worin das genannte Alkyl-β-alkoxyisobutyrat aus der Gruppe, bestehend aus Methyl-β-methoxyisobutyrat, Ethyl-β-methoxyisobutyrat, Methyl-β-ethoxyisobutyrat, Ethyl-β-ethoxyisobutyrat, Methyl-β-isopropoxyisobutyrat, Ethyl-β-isopropoxyisobutyrat, Butyl-β-isopropoxyisobutyrat, Methyl-β-butoxyisobutyrat, Ethyl-β-butoxyisobutyrat und Butyl-β-butoxyisobutyrat, ausgewählt ist.

## Revendications

1. Utilisation d'une composition comprenant comme composé actif au moins un β-alcoxyisobutyrate d'alkyle représenté par la formule (I): 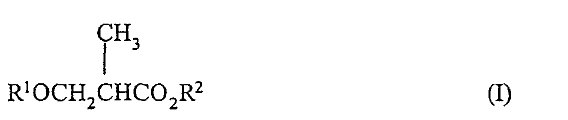 où R¹ et R² représentent chacun un groupe alkyle ayant de 1 à 4 atomes de carbone, dans une quantité d'au moins 5 % en poids, comme solvant.

2. Utilisation selon la revendication 1, dans laquelle ladite composition comprend en outre au moins un composé choisi parmi l'eau, le méthanol, l'éthanol, l'alcool isopropylique, le butanol, le méthylisobutylcarbinol, l'heptanol, l'octanol, le nonanol, le 3-méthylbutanol, le propylèneglycol, le 3-méthoxybutanol, le 3-méthyl-3-méthoxybutanol, le 3,5,5-triméthyl-1-hexanol, le 2-éthyl-1-hexanol, le cyclohexanol, l'alcool benzylique, l'acétone, la méthyléthylcétone, la méthylisobutylcétone, la cyclohexanone, l'isophorone, la pyrrolidone, la N-méthylpyrrolidone, le diméthylformamide, le diméthylacétamide, le diméthylsulfoxyde, le sulfolane, l'acétate de méthyle, l'acétate d'éthyle, l'acétate de butyle, l'acétate d'amyle, l'acétate de 3-méthoxybutyle, l'acétate de 3-méthyl-3-méthoxybutyle, le lactate de méthyle, le lactate d'éthyle, le lactate de butyle, le 3-méthoxypropionate de méthyle, le 3-éthoxypropionate d'éthyle, l'acétate de 2-éthylhexyle, l'acétate de cyclohexyle, l'acétate de benzyle, le dibenzyléther, le nitrométhane, le nitroéthane, l'acétonitrile, la γ-butyrolactone, l'acétate de monométhyléther de propylèneglycol, le toluène, le xylène, l'hexane et le cyclohexane.

3. Utilisation selon la revendication 1, dans laquelle ledit β-alcoxyisobutyrate d'alkyle est choisi parmi le β-méthoxyisobutyrate de méthyle, le β-méthoxyisobutyrate d'éthyle, le β-éthoxyisobutyrate de méthyle, le β-éthoxyisobutyrate d'éthyle, le β-isopropoxyisobutyrate de méthyle, le β-isopropoxyisobutyrate d'éthyle, le β-isopropoxyisobutyrate de butyle, le β-butoxyisobutyrate de méthyle, le β-butoxyisobutyrate d'éthyle et le β-butoxyisobutyrate de butyle.
